# EUROPEAN PATENT SPECIFICATION

(11) **EP 2 235 134 B1**
(45) Date of publication and mention of the grant of the patent: **04.07.2018**
(21) Application number: 08864455.4
(22) Date of filing: 19.12.2008
(51) Int. Cl.: A01N 25/30, A01N 57/20, C07C 233/36

(54) **PESTICIDAL COMPOSTION COMPRISING AN AMIDOAMINE ALKOXYLATE AS ADJUVANT**
PESTIZID-ZUBEREITUNG ENTHALTEND EIN AMIDOAMIN-ALKOXYLAT ALS ADJUVANS
COMPOSITION PESTICIDE COMPRENANT AN AMIDOAMINE ALKOXYLATE COMME ADJUVANT

(30) Priority: 21.12.2007 US 16187
(43) Date of publication of application: 06.10.2010
(73) Proprietor: Huntsman Petrochemical LLC, The Woodlands, TX 77380 (US)
(72) Inventor: STERN, Alan J., Magnolia TX 77354 (US); ELSIK, Curtis, M., The Woodlands TX 77382 (US)
(74) Representative: Van den Broeck, Kristel Alice
(86) International application number: PCT/US2008/087574
(87) International publication number: WO 2009/082675

(56) References cited:
- WO-A1-01/08482
- WO-A1-98/00392
- WO-A2-98/24758
- US-A- 4 002 785
- US-A- 5 108 628
- US-A- 5 226 943
- US-A1- 2003 087 764
- US-A1- 2006 079 435
- US-B2- 7 241 729

## Description

### BACKGROUND OF THE INVENTION

### Field of the Invention

The present invention is directed to an agricultural composition comprising an adjuvant and a pesticide.

### Background of the Invention

Agricultural formulations typically include active ingredients. To these formulations adjuvants are typically added to enhance the effectiveness of the active ingredients. Adjuvants may be used as surfactants, extenders, wetting agents, sticking agents and fogging agents.

Current adjuvants have many drawbacks. For example, current alkoxylate adjuvants, like tallowamine alkoxylate with 15 ethylene oxide units, work well in low concentration (360 grams acid equivalent per liter (gae/L)) glyphosate-isopropylamine formulations, but are unable to be used effectively in high concentration glyphosatepotassium salts.

WO 01/08482 A1 relates to concentrated herbicidal compositions comprising water-soluble glyphosate salts and a surfactant based on a specifically defined alkyl and/or alkenyl amidoamine oxide.

Thus, efforts are continually being made to define new and improved agricultural adjuvants and methods and processes of making them in order to improve cost, handling, compatibility, and/or other properties of such adjuvants.

### BRIEF SUMMARY OF SOME OF THE PREFERRED EMBODIMENTS

In one embodiment of the present invention, an agricultural composition comprising an adjuvant and a pesticide is disclosed, the adjuvant comprising an amidoamine alkoxylate having the formula: 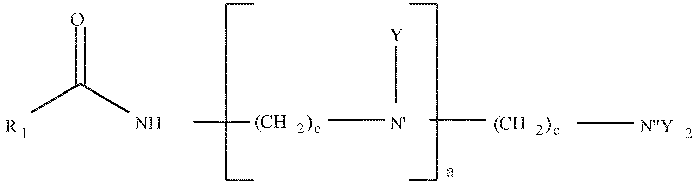 wherein a = 1-3; c = 2-3; R₁ = C₅ to C₁₉ alkyl radical and wherein Y is each independently: H, or 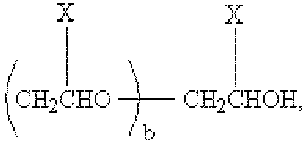 and at least one Y is 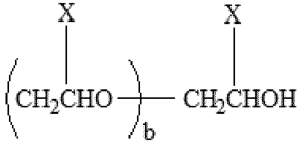 wherein X is each independently H, CH₃ or C₂H₅, b = 0- 10.

### DETAILED DESCRIPTION OF THE PREFERRED EMBODIMENTS

In an embodiment of the present invention, an agricultural composition comprising an adjuvant and a pesticide is disclosed, the adjuvant comprising an amidoamine alkoxylate composition. The amidoamine alkoxylate has the formula: 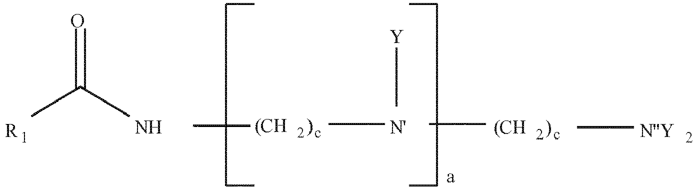 wherein a = 1-3; c = 2-3; R₁ = C₅ to C₁₉ alkyl radical and wherein Y is each independently: H, or 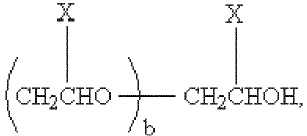 and at least one Y is 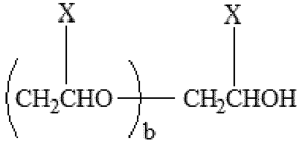 wherein X is each independently H, CH₃ or C₂H₅, b = 0- 10.

In embodiments of the present invention, at least one of N' and N" is quaternized. In another embodiment of the present invention, at least one of N' and N" has been oxidized to form an amine oxide. One skilled in the art, with the benefit of this disclosure, will recognize other possible variations of the above composition.

Such an amidoamine alkoxylate may find beneficial use in numerous applications, including without limitation, agrichemicals, coatings, polymers, resins, fuels, lubes, process additives, and gas treating. One skilled in the art, with the benefit of this application would recognize a suitable application for this amidoamine alkoxylate.

The amidoamine alkoxylate above may be produced by the following method. The sequence below shows how the intermediate amidoamine is made from a triglyceride by reaction with an alkyleneamine.

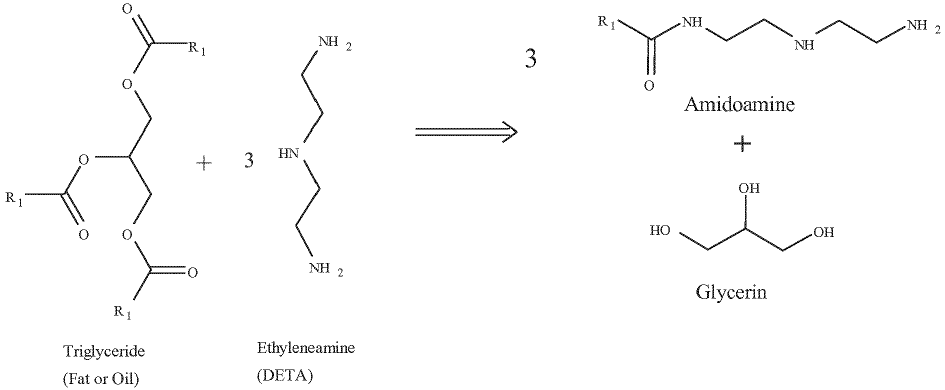

The triglyceride can be selected from oils or fats. Suitable triglycerides may be obtained from both plant and animal sources. Coconut oil, canola oil and palm oil may be used as the triglyceride. R₁ in the triglyceride above may be a fatty alkyl group. Furthermore, fatty acids could be used in place of fats and oils, with some modifications to the process. In another embodiment, a carboxylic acid, such as 2-ethylhexanoic acid may be used in place of the triglyceride. In another embodiment, a combination of triglyceride and carboxylic acid may be used to react with the alkyleneamine. One skilled in the art, with the benefit of this disclosure, would recognize appropriate triglycerides, carboxylic acids and combinations thereof for use in embodiments of the present invention.

The alkyleneamine may be selected from various amines, including ethyleneamine. Such ethyleneamines may include diethylenetriamine (DETA), tetraethylenetriamine (TETA), and tetraethylene pentamine (TEPA). DETA is shown in the sequence above. Dipropylene triamine may also be used. One skilled in the art, with the benefit of this disclosure, would recognize appropriate alkyleneamines for use in embodiments of the present invention.

For the above reaction, it is recommended to have at a minimum the molar ratio of triglyceride to alkyleneamine of 1:3 or about 1:3, however, it is preferable to use an excess of the alkyleneamine component. According to an embodiment, the molar ratio of triglyceride to alkyleneamine may be greater than 1:3 favoring the alkyleneamine.

The intermediate product mixture contains the amidoamine plus glycerin, and other minor products (not shown). It is recognized that some diamide will also form during the reaction. Diamide formation may be desirable when the alkyleneamine is TETA or TEPA. The amount of diamide may vary depending on the ratios of triglyceride to alklyeneamine used.

The amidoamine is then alkoxylated with an alkylene oxide, such as ethylene oxide, as shown below. In another embodiment, the alkylene oxide may include propylene oxide. One skilled in the art, with the benefit of this disclosure, would recognize other appropriate alkylene oxides for use in embodiments of the present invention.

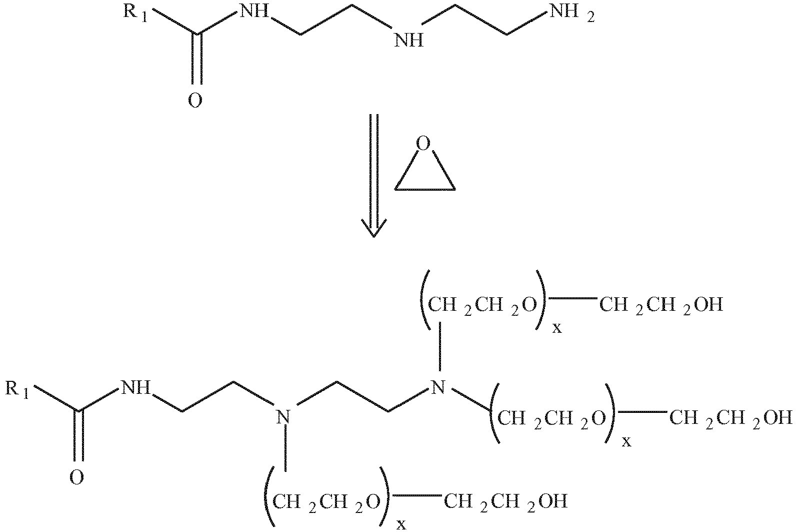

In this structure, x can be from 0 to 5, preferably 0 to 2

Once formed, the amidoamine alkoxylate may be used alone or combine with other chemicals for use in a variety of applications. The amidoamine alkoxylate is used as the adjuvant in the agricultural composition of the present invention.

The pesticide in the agricultural composition of the present encompasses all agriculturally active ingredients and combinations of such active ingredients. In an embodiment of the present invention the pesticide is a herbicide.

In one embodiment of the present invention, the pesticide is glyphosate. In another embodiment of the present invention, the pesticide is glufosinate and salts thereof. In another embodiment, the pesticide is a combination of glyphosate and glufosinate. Any agriculturally acceptable glyphosate (or glufosinate) salt can be used, but the most preferred salts are isopropylamine salts, potassium salts, and ammonium salts. Generally, a high concentration of glyphosate salt (or glufosinate salt) is desired, as long as the resulting blend is homogenous, has a suitable viscosity, and is physically stable over the temperature range -20°C to 60°C. High concentrations of glyphosate may be considered about 450 grams acid equivalent per liter (gae/L) ae and above. Another high concentration that is typically used is about 540 gae/L. A high glyphosate concentration example can be made from 58% potassium glyphosate solution (84 parts), water (8 parts), and adjuvant (8 parts). This formulation contains 540 gae/L glyphosate acid. More or less of each component will also work.

According to an embodiment of the present invention, the pesticide may have a concentration of at least about 540 gae/L. According to an embodiment of the present invention, the pesticide may have a concentration of at least about 600 gae/L.

According to an embodiment of the present invention, the pesticide may comprise an acid salt. According to an embodiment of the present invention, the acid salt may be selected from the group consisting of: a potassium salt, an isopropylamine salt, and an ammonium salt.

Useful ranges for the amidoamine alkoxylate in glyphosate salt aqueous formulations may be from about 1% to about 25% on a weight basis. However, the amount of amidoamine alkoxylates included, for example, in the weed killing formulation, will depend on several factors such as the amount of glyphosate present.

To the agricultural composition described above other active ingredients, additives and solvents may be added. One skilled in the art would recognize appropriate active ingredients, additives and solvents that may be combined with this agricultural composition.

Also disclosed is a method of killing or controlling pests. The agricultural composition listed above is contacted with the pest. The agricultural composition may be used in the above listed for or diluted with water or an appropriate diluent.

### EXAMPLES

Example 1: Synthesis of amidoamine alkoxylate. In a 2 liter, 3 neck round bottom flask equipped with a nitrogen inlet and overhead mechanical agitation was placed 952 grams of molten coconut oil (Columbus Foods Company of Chicago, Illinois) and 448 grams of diethylenetriamine (Huntsman Corporation). Under a nitrogen blanket, this was stirred and heated for 4 hours at 150°C. The intermediate product (called the amidoamine) solidified to an oily yellow paste upon cooling. A 976 gram portion of this intermediate product was transferred to a reactor for ethoxylation. Under a nitrogen blanket, the amidoamine was heated to 150°C and the reactor charged with 803 grams of ethylene oxide (EO) over a one hour period. During the EO addition, the reaction temperature was allowed to rise to 160°C. When the ethylene oxide addition was complete, the reactor was maintained at 150°C for an additional 2.5 hours to digest any remaining EO. It was then cooled to 115°C and discharged from the reactor, yielding a thick, clear amber liquid.

Example 2: Synthesis of amidoamine alkoxylate. In a 2 liter, 3 neck round bottom flask equipped with a nitrogen inlet and overhead mechanical agitation was placed 896 grams of molten coconut oil (Columbus Foods Company of Chicago, Illinois) and 504 grams of diethylenetriamine (Huntsman Corporation). Under a nitrogen blanket, this was stirred and heated for 4 hours at 150°C. The intermediate product (called the amidoamine) solidified to an oily yellow paste upon cooling. A 976 gram portion of this intermediate product was transferred to a reactor for ethoxylation. Under a nitrogen blanket, the amidoamine was heated to 150°C and the reactor charged with 944 grams of EO over a one hour period. During the EO addition, the reaction temperature was allowed to rise to 160°C. When the EO addition was complete, the reactor was maintained at 150°C for an additional 2.5 hours to digest any remaining EO. It was then cooled to 115°C and discharged from the reactor, yielding a thick, clear amber liquid.

Example 3: Synthesis of amidoamine alkoxylate. In a 2 liter, 3 neck round bottom flask equipped with a nitrogen inlet and overhead mechanical agitation was placed 840 grams of molten coconut oil (Columbus Foods Company of Chicago, Illinois) and 560 grams of diethylenetriamine (Huntsman Corporation). Under a nitrogen blanket, this was stirred and heated for 4 hours at 150°C. The intermediate product (called the amidoamine) solidified to an oily yellow paste upon cooling. A 976 gram portion of this intermediate product was transferred to a reactor for ethoxylation. Under a nitrogen blanket, the amidoamine was heated to 150°C and the reactor charged with 1076 grams of EO over a one hour period. During the EO addition, the reaction temperature was allowed to rise to 160°C. When the ethylene oxide addition was complete, the reactor was maintained at 150°C for an additional 2.5 hours to digest any remaining EO. It was then cooled to 115°C and discharged from the reactor, yielding a thick, clear amber liquid.

For the above reactions, appropriate ranges for the alkyleneamine (A), the triglyceride (B), and alkylene oxide (C) can be expressed in terms of weight ratios. When A is coconut oil and B is DETA, the preferred ratio of A to B is from 3:1 to 1:1. In examples 1-3, the ratios of A:B:C are as follows in Table 1.

**Table 1: Ratios of alkyleneamine, triglyceride and alkylene oxide**

| Example | A (DETA) | B (Coconut oil) | C (Ethylene oxide) |
|---|---|---|---|
| 1 | 18 | 37 | 45 |
| 2 | 18 | 33 | 49 |
| 3 | 19 | 29 | 52 |

Examples 4 - 10 show agricultural formulations of amidoamine alkoxylate and glyphosate salts. The percentages refer to weight percent.

### Example 4.

| Component | Percent |
|---|---|
| Amidoamine Alkoxylate from Example 1 | 8 |
| Water | 8 |
| 58% potassium glyphosate liquid | 84 |

Result: a clear, homogenous, fluid yellow liquid; 540 gae/L.

### Example 5.

| Component | Percent |
|---|---|
| Amidoamine Alkoxylate from Example 1 | 10 |
| Water | 23.9 |
| 62% glyphosate-isopropylamine salt liquid | 66.1 |

Result: a clear, homogenous, fluid yellow liquid; 360 gae/L.

### Example 6.

| Component | Percent |
|---|---|
| Amidoamine Alkoxylate from Example 2 | 8 |
| Water | 8 |
| 58% potassium glyphosate liquid | 84 |

Result: a clear, homogenous, fluid yellow liquid; 540 gae/L.

### Example 7.

| Component | Percent |
|---|---|
| Amidoamine Alkoxylate from Example 2 | 10 |
| Water | 23.9 |
| 62% glyphosate-isopropylamine salt liquid | 66.1 |

Result: a clear, homogenous, fluid yellow liquid; 360 gae/L.

### Example 8.

| Component | Percent |
|---|---|
| Amidoamine Alkoxylate from Example 3 | 8 |
| Water | 8 |
| 58% potassium glyphosate liquid | 84 |

Result: a clear, homogenous, fluid yellow liquid; 540 gae/L.

### Example 9.

| Component | Percent |
|---|---|
| Amidoamine Alkoxylate from Example 3 | 10 |
| Water | 23.9 |
| 62% glyphosate-isopropylamine salt liquid | 66.1 |

Result: a clear, homogenous, fluid yellow liquid; 360 gae/L.

### Example 10.

| Component | Percent |
|---|---|
| Amidoamine Alkoxylate from Example 2 | 10 |
| Water | 0 |
| 58% potassium glyphosate liquid | 90 |

Result: a clear, homogenous, fluid yellow liquid, density = 1.407 g/ml at 22 C; 600 gae/L.

Examples 4, 6, 8 and 10 show the amidoamine alkoxylate is surprisingly compatible with highly concentrated potassium glyphosate solutions. The most concentrated known liquid potassium glyphosate formulation in the prior art is 540 gae/L of glyphosate. Using the new adjuvant, 600 gae/L is easily achieved.

Examples 5, 7, and 9 show the amidoamine alkoxylate is compatible with highly concentrated isopropylamine glyphosate solutions.

Surprisingly, Examples 4, 6, 8 and 10 show that with the new amidoamine alkoxylate it is possible to make potassium glyphosate liquid formulations that are fluid (low viscosity) and highly concentrated.

The new amidoamine alkoxylate are also very electrolyte tolerant, if in their protonated (cationic) form. Thus, it is possible to make a blend of ammonium sulfate, acetic acid, and the surfactant in water at high concentration. Example 11 shows such a blend. Other acids will work in place of acetic acid. The percentages refer to weight percent.

### Example 11.

| Component | Percent |
|---|---|
| Ammonium sulfate | 20 |
| Glacial acetic acid | 5 |
| Amidoamine Alkoxylate from Example 1 | 25 |
| Water | 50 |

Result: a clear, homogenous, fluid yellow liquid.

Bioefficacy of the amidoamine alkoxylate with glyphosate. A formulation of glyphosate and the amidoamine alkoxylate was successfully tested in field trials and showed positive results. The plant species in the test were: tall waterhemp, velvetleaf, ivyleaf, morningglory, common cocklebur, and dent corn.

Example 12: Synthesis of amidoamine alkoxylate. In a 2 liter, 3 neck round bottom flask equipped with a nitrogen inlet and overhead mechanical agitation was placed canola oil, 1000 grams, and diethylenetriamine (Huntsman Corporation), 500 grams. Under a nitrogen blanket, this was stirred and heated for 90 minutes at 160°C. The reaction mixture (called the amidoamine) was cooled to 80°C. The amidoamine was transferred to a reactor for ethoxylation. After purging the reactor with nitrogen gas, the amidoamine was heated to 140°C and the reactor charged with 1.5 kg of ethylene oxide over a 90 minute period. During the EO addition, the reaction temperature was allowed to rise to 147°C. When the ethylene oxide addition was complete, the reactor was maintained at 145°C for an additional 2.0 hours to digest any remaining EO. It was then cooled to 115°C and discharged from the reactor, yielding a thick, clear amber liquid.

Example 13. Synthesis of amidoamine alkoxylate from a carboxylic acid. In a 2 liter, 3 neck round bottom flask equipped with a nitrogen inlet and overhead mechanical agitation and a Dean-Stark water collector was placed 2-ethylhexanoic acid, 672 grams, (Sigma-Aldrich, Inc. of St. Louis, Missouri) and diethylenetriamine (Huntsman Corporation), 600 grams. This was stirred and heated for 2 hours at 180°C and then for 2 hours at 185°C. During this time, 93 grams of clear liquid was collected in the Dean-Stark apparatus. The reaction mixture (called the amidoamine) was cooled to 80°C and 1.1 kg was transferred to a reactor for ethoxylation. After purging the reactor with nitrogen gas, the amidoamine was heated to 147°C and the reactor charged with 0.9 kg of ethylene oxide over a 90 minute period. When the ethylene oxide addition was complete, the reactor was maintained at 147°C for an additional 2.0 hours to digest any remaining EO. It was then cooled to 115°C and discharged from the reactor, yielding a thick, clear amber liquid.

Example 14. Synthesis of amidoamine alkoxylate. In a 2 liter, 3 neck round bottom flask equipped with a nitrogen inlet and overhead mechanical agitation was placed coconut oil 603.5 grams (Columbus Foods Company of Chicago, Illinois) and dipropylene triamine, 510 grams (Sigma-Aldrich, Inc. of St. Louis, Missouri). Under a gentle nitrogen stream, this was stirred and heated for 4.5 hours at 160°C. The reaction mixture (called the amidoamine) was cooled to 80°C and 1.0 kg was transferred to a reactor for ethoxylation. After purging the reactor with nitrogen gas, the amidoamine was heated to 160°C and the reactor charged with 1.0 kg of ethylene oxide over a 90 minute period. When the ethylene oxide addition was complete, the reactor was maintained at 160°C for an additional 2.0 hours to digest any remaining EO. It was then cooled to 115°C and discharged from the reactor, yielding a thick, clear amber liquid.

Examples 15 - 23 show agricultural formulations of amidoamine alkoxylate and glyphosate salts. The percentages refer to weight percent.

### Example 15.

| Component | Percent |
|---|---|
| Adjuvant from example 12 | 8 |
| Water | 7 |
| 58% potassium glyphosate liquid | 85 |

Result: a clear, homogenous, fluid yellow liquid at 25°C.
At 50 °C, this blend separated into two phases.

### Example 16.

| Component | Percent |
|---|---|
| Adjuvant from example 12 | 10 |
| Water | 23.9 |
| 62% glyphosate-isopropylamine salt liquid | 66.1 |

Result: a clear, homogenous, fluid amber-colored liquid at 21°C.

### Example 17.

| Component | Percent |
|---|---|
| Adjuvant from example 13 | 8 |
| Water | 7 |
| 58% potassium glyphosate liquid | 85 |

Result: a clear, homogenous, fluid yellow liquid at 21 °C.

### Example 18.

| Component | Percent |
|---|---|
| Adjuvant from example 13 | 10 |
| Water | 23.9 |
| 62% glyphosate-isopropylamine salt liquid | 66.1 |

Result: a cloudy, inhomogenous mixture at 21°C.

### Example 19.

| Component | Percent |
|---|---|
| Adjuvant from example 13 | 25 |
| Glufosinate solution, 50% active | 36.1 |
| Propylene glycol methyl ether | 10 |
| Acetic acid, glacial | 7.2 |
| Water | 21.7 |

Result: a clear, homogenous, fluid yellow liquid at 21°C.

### Example 20.

| Component | Percent |
|---|---|
| Adjuvant from example 14 | 8 |
| Water | 7 |
| 58% potassium glyphosate liquid | 85 |

Result: a clear, homogenous, fluid yellow liquid at 25°C.

### Example 21.

| Component | Percent |
|---|---|
| Adjuvant from example 14 | 10 |
| Water | 23.9 |
| 62% glyphosate-isopropylamine salt liquid | 66.1 |

Result: a clear, homogenous, fluid amber-colored liquid at 25°C.

### Example 22.

| Component | Percent |
|---|---|
| Adjuvant from example 14 | 25 |
| Glufosinate solution, 50% active | 36.1 |
| Propylene glycol methyl ether | 10 |
| Acetic acid, glacial | 7.2 |
| Water | 21.7 |

Result: a clear, homogenous, fluid yellow liquid at 25°C.

### Example 23.

| Component | Percent |
|---|---|
| Adjuvant from example 13 | 25 |
| Glufosinate solution, 50% active | 65 |
| Acetic acid, glacial | 10 |

Result: a clear, homogenous, viscous yellow liquid at 21°C.

Example 24. Oxidation of an amidoamine alkoxylate with hydrogen peroxide. The amidoamine alkoxylate of Example 12 (130 grams) was placed in a flask equipped with a mechanical stirrer. With continuous stirring, Versene 100 chelating agent (1 gram), (The Dow Chemical Company, Midland, Michigan) was added. Next, hydrogen peroxide (42 grams of a 35% peroxide solution in water) was added in small portions over a 120 minute period, taking care to keep the temperature of the reaction between 35 °C and 60 °C. After the peroxide addition was complete, the mixture was stirred for an additional 120 minutes. The product is a viscous yellow liquid.

Examples 25 - 27 show pesticide formulations using the oxidized amidoamine alkoxylate. The percentages refer to weight percent.

### Example 25.

| Component | Percent |
|---|---|
| Adjuvant from Example 24 | 10 |
| Water | 5 |
| 58% potassium glyphosate liquid | 85 |

Result: a clear, homogenous, slightly viscous, light yellow liquid

### Example 26.

| Component | Percent |
|---|---|
| Adjuvant from Example 24 | 12 |
| Water | 21.9 |
| 62% glyphosate-isopropylamine salt liquid | 66.1 |

Result: a clear, homogenous, fluid, light yellow liquid at 21°C

### Example 27.

| Component | Percent |
|---|---|
| Adjuvant from Example 24 | 25 |
| Glufosinate ammonium solution, 50% active | 65 |
| Acetic acid, glacial | 10 |

Result: a clear, homogenous, slightly viscous yellow liquid at 21°C.

Example 28. Viscosity of glyphosate salt formulations. The following Table 2 shows viscosity data for some of the example formulations. The data was obtained with a Brookfield DV-II viscometer, equipped with an LV-2 spindle, at 21 °C, at 60 rpm. In the table below, DPT is used as an abbreviation for 3-aminopropyl-1,3-propanediamine. Measurements are in centipoise.

**Table 2: Viscosity Measurements for selected Examples**

| Example | Glyphosate Salt Type | Amidoamine type | cP |
|---|---|---|---|
| 6 | Potassium | Coconut/DET A | 62 |
| 7 | Isopropylamine | Coconut/DETA | 44 |
| 10 | Potassium | Coconut/DET A | 251 |
| 15 | Potassium | Canola/DETA | 167 |
| 16 | Isopropylamine | Canola/DETA | 66 |
| 17 | Potassium | 2-ethylhexanoic/DETA | 70 |
| 18 | Isopropylamine | 2-ethylhexanoic/DETA | 43 |
| 20 | Potassium | Coconut/DPT | 66.5 |
| 21 | Isopropylamine | Coconut/DPT | 43 |

## Claims

1. An agricultural composition comprising an adjuvant and a pesticide, the adjuvant comprising an amidoamine alkoxylate of the formula: 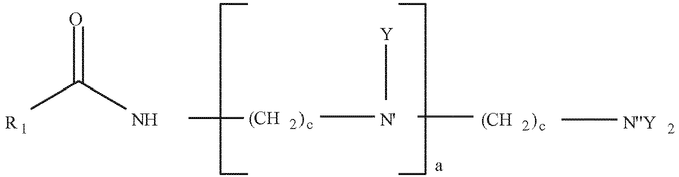 wherein a = 1-3; c = 2-3; R₁ = C₅ to C₁₉ alkyl radical, wherein Y is each independently: H, or 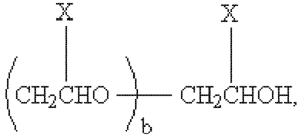 and at least one Y is 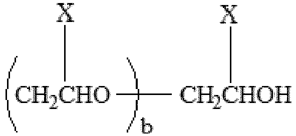 wherein X is each independently H, CH₃ or C₂H₅, b = 0-10 .

2. The composition of claim 1 wherein at least one of N' or N" is quaternized.

3. The composition of claim 1 wherein at least one of N' or N" is oxidized.

4. A composition of claim 1 wherein the pesticide is selected from the group consisting of: a glyphosate, a glufosinate and a combination thereof.

5. A composition of claim 4 wherein the pesticide has a concentration of at least about 540 gae/L.

6. A composition of claim 1 wherein the pesticide comprises an acid salt.

7. A composition of claim 6 wherein the acid salt is selected from the group consisting of: a potassium salt, an isopropylamine salt, and an ammonium salt.

## Patentansprüche

1. Landwirtschaftliche Zusammensetzung, umfassend ein Adjuvans und ein Pestizid, wobei das Adjuvans ein Amidoaminalkoxylat der Formel: 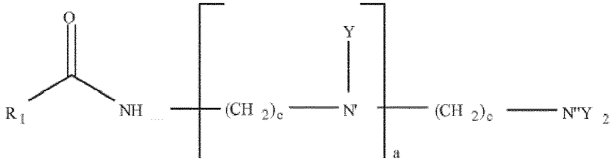 umfasst, worin a = 1-3; c = 2-3; R₁ = C₅- bis C₁₉-Alkylrest, worin Y jeweils unabhängig H oder 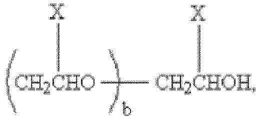 ist und mindestens ein Y 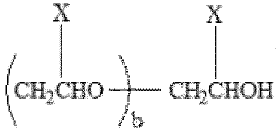 ist, worin X jeweils unabhängig H, CH₃ oder C₂H₅ ist, b = 0-10.

2. Zusammensetzung nach Anspruch 1, wobei mindestens eines von N' oder N" quaternisiert ist.

3. Zusammensetzung nach Anspruch 1, wobei mindestens eines von N' oder N" oxidiert ist.

4. Zusammensetzung nach Anspruch 1, wobei das Pestizid ausgewählt ist aus der Gruppe bestehend aus: einem Glyphosat, einem Glufosinat und einer Kombination davon.

5. Zusammensetzung nach Anspruch 4, wobei das Pestizid eine Konzentration von mindestens etwa 540 gae/L aufweist.

6. Zusammensetzung nach Anspruch 1, wobei das Pestizid ein Säuresalz umfasst.

7. Zusammensetzung nach Anspruch 6, wobei das Säuresalz ausgewählt ist aus der Gruppe bestehend aus: einem Kaliumsalz, einem Isopropylaminsalz und einem Ammoniumsalz.

## Revendications

1. Composition agricole comprenant un adjuvant et un pesticide, l'adjuvant comprenant un alkoxylate d'amidoamine de formule : 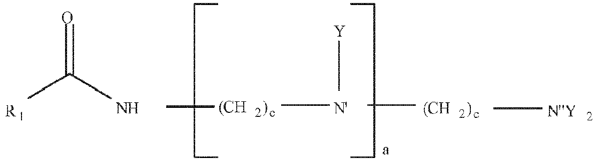 dans laquelle a = 1 à 3 ; c = 2 à 3 ; R₁ = un radical alkyle en C₅ à C₁₉, dans laquelle Y est chacun indépendamment : H, ou 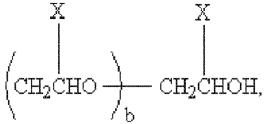 et au moins un Y est 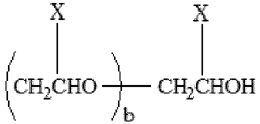 X étant chacun indépendamment H, un groupe CH₃ ou C₂H₅, b = 0 à 10.

2. Composition selon la revendication 1, dans laquelle au moins un de N' ou N'' est quaternarisé.

3. Composition selon la revendication 1, dans laquelle au moins un de N' et N'' est oxydé.

4. Composition selon la revendication 1, dans laquelle le pesticide est choisi dans le groupe constitué par : un glyphosate, un glufosinate et une de leurs combinaisons.

5. Composition selon la revendication 4, dans laquelle le pesticide a une concentration d'au moins environ 540 gae/l.

6. Composition selon la revendication 1, dans laquelle le pesticide comprend un sel acide.

7. Composition selon la revendication 6, dans laquelle le sel acide est choisi dans le groupe constitué par : un sel de potassium, un sel d'isopropylamine et un sel d'ammonium.
